(19) Europäisches Patentamt
European Patent Office
Office européen des brevets

(11) Publication number: **0 158 490**
**A2**

(12) # EUROPEAN PATENT APPLICATION

(21) Application number: 85302221.8

(22) Date of filing: 29.03.85

(51) Int. Cl.⁴: A 61 F 13/18
A 41 B 13/02

(30) Priority: 30.03.84 US 595184
30.03.84 US 595185
30.03.84 US 595206

(43) Date of publication of application:
16.10.85 Bulletin 85/42

(84) Designated Contracting States:
AT BE CH DE FR GB IT LI LU NL SE

(71) Applicant: PERSONAL PRODUCTS COMPANY
Van Liew Avenue
Milltown New Jersey 08850(US)

(72) Inventor: Korpman, Ralf
885 Bluestone Lane
Somerset, NJ 08807(US)

(74) Representative: Jones, Alan John et al,
CARPMAELS & RANSFORD 43 Bloomsbury Square
London, WC1A 2RA(GB)

(54) **Extruded absorbent article.**

(57) A disposable absorbent structure is provided by an initially molten film, generally extruded, an absorbent unit and fabric. The components are laminated and provide a highly stable, flexible and soft product which contains at least about 5 mls of body exudate per gram.

FIG-1

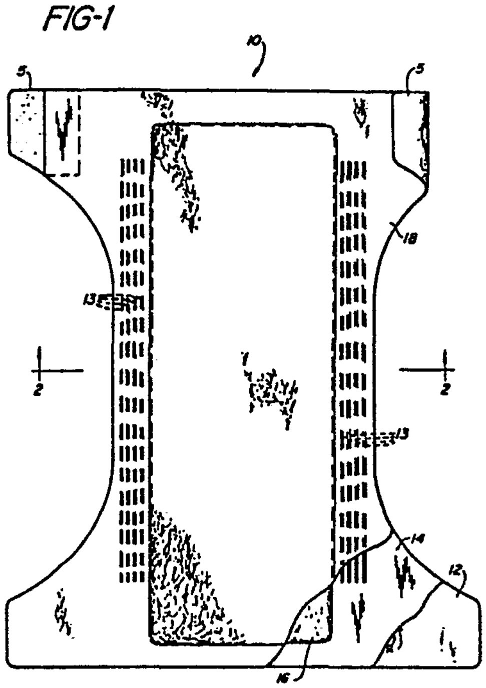


EP 0 158 490 A2

Croydon Printing Company Ltd.

## Extruded Absorbent Article

### Background of the Invention

The present invention relates to an extruded absorbent article which is a disposable article such as disposable diapers, sanitary napkins, incontinent pads, and the like. The absorbent article includes an initially molten film having laminated thereto an absorbent unit and a fabric.

Disposable diapers, sanitary napkins, incontinent pads and similar disposable absorbent articles have become commercially important. The structure of these articles is similar and generally has three basic elements: a liquid-impermeable backing, an absorbent unit, and a liquid-permeable facing. Generally, the backing is of a film such as a polyethylene film. Typical prior art diapers are shown in U.S. Patent Nos. Re. 26,151 and 3,612,055. Generally, the absorbent unit is smaller in size than the backing or the facing and is sandwiched between the backing and facing, the latter two being laminated one to the other in the marginal portions. Frequently, the absorbent unit is a fluff batt of loosely compacted cellulosic fibers, such as wood pulp fibers.

The backing film, typically a polyethylene film, though it is liquid-impermeable, is frequently noisy and creates a "greenhouse" effect. The greenhouse effect is caused by the non-breathability of the polyethylene film. Lack of breathability causes skin irritation when the diaper product becomes wet and the moisture vapors are unable to escape. The slick feel of the polyethylene film backing is another drawback to the conventional disposable diaper.

Many attempts have been made to overcome these drawbacks. For instance, a diaper having a fibrous outer layer is shown in U.S. Patent No. 3,779,246. While diapers formed in accordance with this patent have improved feel and appearance as compared to diapers having an exposed plastic sheet as the outermost layer, the additional fabric layer coupled with the necessary adhesive introduces additional thickness which in turn can impair flexibility.

Another problem with commercially available disposable diapers is the stability of the fluff batt. Many improvements have been made to stabilize the fluff batt. For instance in U.S. Patent No. 3,017,304 a paper-like densified skin is provided on the outermost side of the fluff batt, and in U.S. Patent 3,938,522 densified regions, generally in the form of embossed lines, are present to both stabilize the fluff batt and to promote wicking. Whereas these techniques resulted in an improved product, the paper-like densified skin and/or the densified regions tend to stiffen the fluff batt and it is known that the densified regions are of reduced liquid holding capacity.

All of the known disposable products are laminated products wherein adhesive or glue is required to adhere one layer of the product to another. The adhesive or glue also lends thickness to the product, and hence can make the product less flexible. Furthermore, the adhesive or glue detracts from the absorbent capacity of the product.

An attempt was made to produce a glueless product in U.S. Patent No. 2,897,108. Tissue sheets were arranged in a stepped fashion symmetrically one above the other, whereby marginal areas of each successive tissue sheet extended beyond the counterpart edge of the next smaller

BAD ORIGINAL

tissue sheet. A continuous liquid-impermeable plastic film is extrusion coated on to the stacked tissue sheets so as to be coextensive in area with the largest tissue sheet. The film extends across and in contact with the outwardly facing surface of the smallest tissue sheet and the successive marginal areas of the larger tissue sheets and is bonded at least at selected areas to each of the contact tissue sheets. The articles produced by the patent have not met with commercial success for a variety of reasons. First of all, the article is so thin as to be fragile. Second, absorbency is entirely inadequate because the tissue sinks into and becomes integral with the film causing substantial loss of potential absorbency. In essence, the product formed by the patent is a sheet of conventional commercially available tissue to which a thin coating of plastic material is applied. The paper-like feel and extremely limited absorptive capacity renders such a product unacceptable as a disposable absorbent product in today's marketplace.

There remains the unsolved need in the marketplace for a product having absorbency characteristics at least equal to those of presently available disposable products and also a product which can be produced by less complex and more inexpensive processing and equipment.

## Summary of the Invention

The present invention provides an absorbent structure which contains an initially molten film having laminated thereto an absorbent unit on one side thereof and preferably a fabric laminated to and substantially coterminous with the film. This absorbent structure is capable of absorbing liquid in an amount of at least about five milliliters per gram of product weight.

The initially molten film may be a pressure-sensitive adhesive film, or an ordinary thermoplastic film, or an elastomeric thermoplastic film, or mixtures thereof.

The absorbent unit of the present invention is a fluff batt or a fabric associated with superabsorbent or a self-contained unit or the like. The optional fabric of the present invention is a liquid-permeable fabric or film such as a perforated polyethylene film, or a nonwoven fabric such as a polyester or the like when used on the side covering the absorbent unit. Any soft fabric is suitable for use on the outside (or back) of the structure.

The absorbent structure of the present invention preferably has a total thickness of at least about 30 mils and a total weight of at least about 6 ounces per square yard.

In a preferred embodiment of the present invention, an initially molten film has a cloth or fabric laminated to one side thereof and on the other side has an absorbent unit smaller in size with a liquid-permeable fabric covering the absorbent unit and coterminous with the film, the liquid-permeable fabric being laminated to the film but on the side opposite the first fabric sheet. Thus, an absorbent structure is provided which is thin, highly flexible, highly absorbent, and optionally has fabric on both sides thereof. Furthermore, the present invention provides a product wherein all components of the product are stabilized and a moisture barrier is provided without the use of glues or adhesives.

Brief Description of the Drawings

Figure 1 is a plan view with portions broken away for clarity of one embodiment of the present invention;

Figure 2 is an enlarged cross-sectional view of the embodiment of Figure 1 taken along line 2-2;

Figure 3 is a plan view with a portion broken away for clarity of another embodiment of the present invention;

Figure 4 is a schematic illustration showing a production line by which an embodiment of the present invention is produced;

Figure 5 is another schematic illustration showing another process by which a still further embodiment of the present invention is prepared;

Figure 6 is a perspective view of a production line for the manufacture of one embodiment of the present invention.

Figure 7 is a perspective view of another embodiment of the present invention;

Figure 8 is a cross-sectional view of yet another embodiment of the present invention.

Figure 9 is a plan view of a corrugated disposable diaper according to the present invention.

Figure 10 is a plan view of a diaper according to the present invention; and

Figure 11 is a plan view of a further diaper according to the present invention.

Detailed Description of the Invention

Referring now to the drawings, Figure 1 illustrates a disposable diaper 10. The disposable diaper 10 has a cloth backing 12 which is a soft, thin fabric such as a nonwoven polyester fabric. The fabric 12 is laminated to the initially molten film 14. The absorbent unit 16 is a fluff batt made from wood pulp fibers and is preformed and then placed on the film 14 while the film is still tacky. Over the absorbent unit side of the product, a liquid-permeable fabric 18 is placed and laminated where the film is exposed in the regions surrounding the absorbent batt 16. The facing fabric 18 is liquid-permeable and may be the same fabric as the backside fabric 12 or it may be different. Perforated films may be substituted for the fabric 18. Before the facing fabric 18 is placed over the product, elastic elements 13 may be added. Closure means 5 are placed in the corners of one end of the diaper so as

to easily secure the product about the waist of the wearer. The closure means 5 may be a portion of film exposed, if the film 14 is a pressure-sensitive adhesive film, or it may be a region wherein a pressure-sensitive adhesive has been placed over the fabric 18. Alternatively, adhesive tape tabs could be added.

Figure 2 is a cross-sectional view showing portion 20 of the diaper of Figure 1 taken along line 2-2 of Figure 1. The fabric backing 22 is totally laminated to the film 24. To the film on the opposite side thereof is placed the absorbent batt 26. Elastic ribbons 23, are in the margin beyond the absorbent batt 26. The liquid-permeable facing 28 is placed over the product on the side opposite the fabric 22 and the fabric 28 is laminated in the side margins where it is in contact with the film 24.

Figure 3 depicts a sanitary napkin manufactured in accordance with the present invention. A portion of the drawing is broken away for clarity. The view is of the bottom side of the product. A fabric 32 normally used for the overwrap of a sanitary napkin 30 is in contact with an initially molten film 36. The initially molten film is coextensive with the fabric 32. An absorbent unit 34 is placed centrally on one side of the film 36 between the film and fabric when the film is still tacky. A window 33 is left in the fabric on the bottom side, when the film is a pressure-sensitive adhesive film, and is covered with a release strip 35 so that the release strip 35 may be removed exposing the adhesive to permit securement of the product in the underwear of the wearer. Alternatively, adhesive lines may be placed on the underside of the product to secure it to the clothing of the wearer.

Figure 4 is a schematic illustration of one method of making an embodiment of the present invention. The

backing fabric 41 is fed to a pair of rolls 43 and 44. A molten pressure-sensitive adhesive film 40 is extruded from a die 42 over the surface of the fabric 41 covering substantially all of the fabric surface. The fabric 41 is laminated to the film 40 on one side of the film 40 by passing through the rolls 43 and 44 leaving the other side of the film open for lamination of other components of the product to the exposed tacky side. Elastic 45, in multiple ribbons, is fed to each side of the product on the exposed surface of film 40 and in between the elastic strips is placed absorbent batt 47, the latter being conveyed to the exposed side of the film. The elastic 45, the absorbent batt 47, and the fabric 46 extending beyond the absorbent batt 47, are laminated by roll 48 to the exposed surface of film 40. The individual diaper products 49 are then severed to provide separate units.

Figure 5 is a schematic illustration of another method which provides another embodiment of the present invention. A fabric 51 is fed to the nip of rolls 53 and 54 and just before encountering the nip of the rolls, a molten film 50 is extruded by a die 52 onto the surface of the fabric 51. Simultaneously with the contact of the molten film and the fabric to the nip of the rolls are fed spaced apart absorbent batts so that the fabric, the molten film, and the absorbent batts 56 come in contact simultaneously. The rolls 53 and 54 laminate the product, and after lamination the conveying belt 55 which conveyed the absorbent batts to the nip of the rolls is taken off in another direction so that the product 57 may be severed and placed into separate units. The product consists of fabric 51 on the under exterior side, film 50, and the absorbent unit 56. In this instance, the absorbent unit 56 should be a self-contained unit which can provide an adequate surface for its intended use.

Figure 6 is a perspective view of a production line for producing multiple units of the product of the present invention. A fabric 61 is fed to a continuously moving conveyor belt. Over the surface of the fabric 61, is placed an extruded molten film 62 which is a pressure-sensitive adhesive film. Elastic strands 63 are fed in spaced arrangement such that between alternate pairs of the elastic strands is placed an absorbent batt 64. In this particular illustration, three absorbent batts are placed simultaneously across the production line in between alternate pairs of elastic ribbons. Next a fabric 65 is placed over the top of the product and the product is laminated with laminating rolls 66. The laminating rolls may be provided with portions of a greater circumference in the regions between the absorbent batts so as to press the fabric, film, and elastic together. Slitters 67 slit the film in between the absorbent batts so as to provide three separate lines of continuous absorbent products. Each line is then divided into separate units by a transverse cutter 68 to provide the individual absorbent product units 69.

Figure 7 illustrates a disposable absorbent structure 70. The structure 70 has an initially molten extruded film layer 72 to which is laminated a cellulosic fibrous batt 74. Because the batt is laminated to the film when the film is in a molten state, the cellulosic fibrous batt is substantially stable. The batt does not tend to break apart. Furthermore, highly satisfactory wicking takes place when liquid is placed in contact with the cellulosic fibrous batt.

Figure 8 is a cross-sectional view of a typical absorbent structure. The absorbent structure 80 consists of a cellulosic fibrous batt 84 and a thin film 82 which when in its molten state was placed in contact with the

cellulosic fibrous batt 84. The film and batt in this instance are coextensive. Fibers 86 from the cellulosic fibrous batt 84 tend to become at least partially embedded in the molten film 82 at the time of lamination. The remainder of the fibers 86 are mechanically entangled with other fibers which may or may not have a portion thereof embedded in the film 82. However, the combination of the mechanical entanglement of the fibers and the embedding of some of the fibers partially into the film creates a substantially, completely stable, cellulosic fibrous batt.

Referring now to Figure 9, a corrugated disposable diaper 110 is illustrated. The view shown in Figure 9 is from the underside of the diaper. However, a portion is broken away for clarity. A liquid-permeable facing 112 extends the entire length and width of the diaper. An absorbent batt 116 is of smaller dimension than the facing 112 so that there is a margin provided surrounding the absorbent batt. An initially molten film 114 is of the same dimension as the facing 112 so that the facing 112 is adhered to the film 114 in the margins surrounding the batt 116. A multiplicity of elastic strings 118, are placed between the molten film 114 and a fabric backing 115. In the embodiment shown in Figure 9, the elastic bands are placed only in the central portion so as to gather and corrugate the absorbent batt in the central portion. Tape tabs 117 are affixed to one end to provide a securement means for the diaper product about the waist of the wearer.

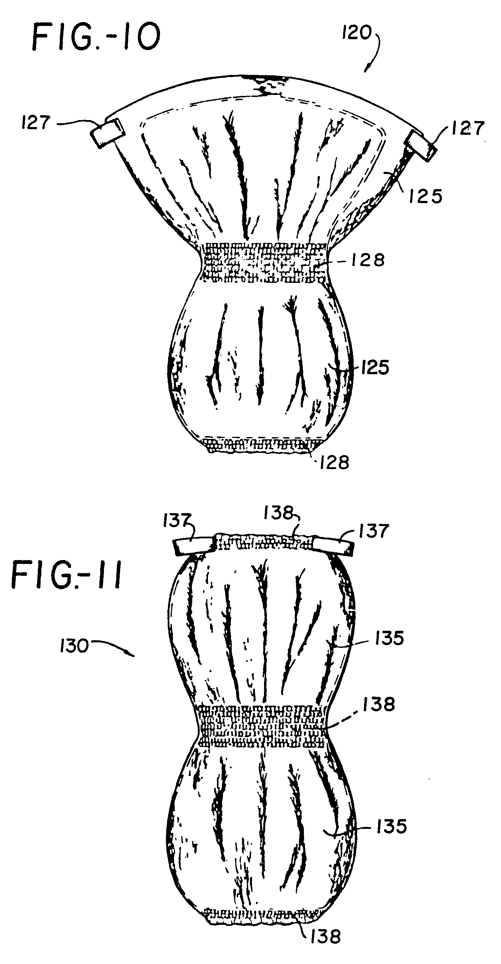

Figure 10 illustrates another embodiment of the present invention wherein a diaper 120 is provided. The components are substantially the same as in Figure 9 except that gathering is provided at regions 128 wherein the diaper is gathered in the central portion and at one

end of the waist portion to provide a bell-like configuration. The cloth backing 125 provides a soft external feel for the diaper product. The tape tabs 127 are affixed to one end to secure the diaper about the waist of the wearer.

In Figure 11, the embodiment differs from Figure 10 in that elastic is provided at both waist portions as well as in the center at regions 138. A cloth-like backing 135 is provided and tape tabs 137 will secure the diaper about the waist of the wearer.

As stated above, articles made by the present invention may be a diaper, a sanitary napkin, an incontinent pad, or the like. The product of the present invention minimally is comprised of an initially molten film, an absorbent unit, and optionally a fabric or film. The initially molten film is extruded to provide a thin tacky film to which the absorbent unit and optional fabric (or film) are laminated while the film is tacky.

Suitable films for providing the initially molten film generally fall into five groups. The first group contains the plastic films which include polyethylene, ethylene-vinyl acetate, polypropylene, polystyrene, polyurethane, polyvinylchloride, polybutylene, co-polyesters, vinyls, and acrylics, and other thermoplastic substances or blends thereof. Suitable polyethylenes are the low and medium density polyethylenes. Homopolymers of polyethylene are suitable but many resins are copolymers containing two to 40 percent vinyl acetate. Thus, an ethylene-vinyl acetate copolymer having a high ethylene content is suitable as a plastic for extrusion to provide the initially molten film. Polypropylene is readily processed at extrusion temperatures of about 400 - 475°F. Extrusion temperatures for the polystyrene thermoplastics may range from 350 -

500°F. Polyurethane is generally extruded as a flexible film or foam. A thin, liquid-impermeable, flexible foam or film of the vinyl copolymers, perhaps the polyvinylchloride, is well known and easy to extrude into the desired, tacky, thin film. Generally, the higher molecular weight resins are used to provide the polyvinylchloride for extrusion.

A suitable copolyester contains at least two identifiable ester units, which may be represented by the formulas:

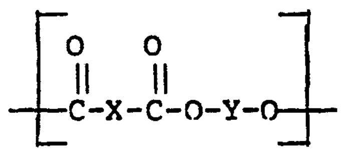

and

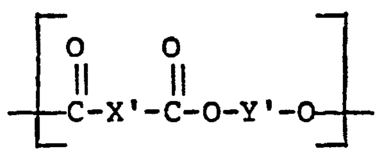

wherein x and x' are nuclei of dicarboxylic acids, and y and y' are nuclei of aliphatic diols.

The different ester units may arise from the condensation of two different acids with the same diol, or two different diols with the same acid, or two different acids and two different diols. By "copolyesters" is meant to include random and segmented copolyesters. By "random" is meant that the ester units are not usually in a definite pattern such as in alternating units, or in having a homopolymer of one ester unit joined terminally to a homopolymer of a second ester unit. By "segmented" is meant that there is a polymer block of a given ester unit joined to a polymer block of another ester unit through an ester linkage. Generally, one ester unit of the copolyester is

highly crystalline and one substantially less crystalline or even non-crystalline.

Among the aromatic and aliphatic dicarboxylic acids suitable in the copolyester are terephthalic acid, isophthalic acid, oxalic acid, maleic acid, succinic acid, glutaric acid, adipic acid, suberic acid, azelaic acid, sebacic acid, and the like. The aliphatic diols useful in the polyester include ethylene glycol, 1,3-propylene glycol, diethylene glycol, dipropylene glycol, butylene glycol, tetramethylene glycol, trimethylene glycol, and the like. Preferred aliphatic diols are straight chain alkylene diols having from two to four carbon atoms.

Representative copolyesters include poly(ethylene terephthalate-co-ethylene azelate), poly(ethylene terephthalate-co-butylene adipate), poly(ethylene terephthalate-co-1,2-propylene terephthalate), poly(butylene terephthalate-co-butylene isophthalate), poly(ethylene terephthalate-co-ethylene sebacate), poly(ethylene terephthalate-co-tetramethylene terephthalate), poly(ethylene terephthalate-co-ethylene adipate), poly(ethylene terephthalate-co-trimethylene terephthalate), and the like.

When the plastic compositions of this first group are used, it is necessary that the film which is extruded be in its molten state at the time that the absorbent unit and fabric come in contact with the film so that the film is still tacky and lamination can take place.

The second group of suitable materials for films which can be extruded to make the products of the present invention are the thermoplastic elastomeric films. One type which is suitable is an ethylene-vinyl acetate film wherein the vinyl acetate content is high, i.e., from about 40 percent to about 60 percent and the ethylene content is low.

Another suitable type is polyurethane. The thermoplastic polyurethane elastomers are prepared from a high molecular weight di-primary diol, a low molecular weight chain extender, and a di-isocyanate. This combination is melt polymerized to form block copolymers with alternating hard and soft segments. The diol is either a polyester or a polyether. Generally, when such a polyurethane is extruded, a single stage extruder is used at temperatures of 320 - 430°F.

Polyolefin blends are also suitable to provide a thermoplastic elastomer. These olefinics are blends of a hard component, generally a crystalline polyolefin such as polypropylene or polyethylene, and a soft portion composed of ethylene-propylene rubber. Generally, these olefinic thermoplastic elastomers are extruded at temperatures of 350 - 450°F.

Some of the aforementioned copolyesters can be found to be elastomeric. Included in these is poly(ethylene terephthalate-co-ethylene azelate). Elastomeric copolyester products can be formed from non-elastomeric copolyesters. For instance, poly(butylene terephthalate-co-butylene isothalate) copolyester composition can be blended in an amount of about 30 percent by weight with about 70 percent by weight of an elastomer. This alloy is then extruded and can be hot drawn to produce the ester modified elastomeric film.

Particularly suitable thermoplastic elastomers are the thermoplastic elastomeric block copolymers having thermoplastic end blocks and rubbery mid blocks which are designated as A-B-A block copolymers. The expression "A-B-A" block copolymer is intended to embrace all variations of block copolymers with rubbery mid blocks and thermoplastic end blocks. It is also intended to embrace radial

block copolymers which may be designated $(A-B)_nX$, wherein X is a poly functional atom or molecule and in which each (A-B) radiates from X in a way that A is an end block. A-B block copolymers sometimes referred to as "simple" block copolymers, in which B forms one end block rather than a mid block, may be included to modify the A-B-A block copolymers and the expression "block copolymer" without qualification is intended to embrace them as well.

The thermoplastic "A" block is generally a polymer of alkenylarenes preferably of styrene or styrene homologues and analogues. The rubbery "B" block is a polymer of a conjugated aliphatic diene of from four to six carbon atoms or a lower alkene of from two to six carbon atoms. Suitable dienes include butadiene, isoprene, and the like. Suitable alkenes include ethylene, butylene, propylene, and the like. In the A-B block copolymers, the B blocks are preferably isoprene. The block copolymers may be linear branched or radial. A branched copolymer is essentially a linear polymer in which branching may occur randomly anywhere in the rubber copolymer chain. A radial block copolymer is characterized and distinguished from the branched linear copolymer in having blocks radiating from a central core.

The individual A block portion has a number average molecular weight of at least 6,000 preferably in the range of from about 8,000 to 30,000, and the B block portion has a number average molecular weight preferably in the range of from about 45,000 to about 180,000. The A block constitute from about five to about 50 percent of the block copolymer. The total block copolymer average molecular weight generally ranges from about 75,000 to about 200,000 for linear copolymers and from about 125,000 to about 400,000 for radial copolymers. In the A-B copolymers, the

total molecular weight generally should not exceed about 150,000.

When the "A" block is polystyrene and the "B" block is a polymer of butadiene in an A-B-A type block copolymer, the polymer is frequently referred to as a S-B-S polymer and when the "A" block is a styrene polymer and the "B" block is an isoprene polymer, the polymer is frequently referred to as a S-I-S polymer. Examples of commercially available block copolymers include Solprene 1205 which is a simple S-B (Phillips Petroleum Company) and Shell Chemical Company's Kraton 1102 which is a linear S-B-S and 1107, 1112, and 1117 which are all linear S-I-S.

The elastomeric component of these thermoplastic elastomers may include small amounts of more conventional elastomers but these should not exceed about 25 percent by weight of the elastomeric component. These elastomers may include highly broken down natural rubbers and butadiene-styrene random copolymer rubbers, synthetic polyisoprene, cloroprene rubbers, nitrile rubbers, butyl rubbers, and the like. Potentially elastomeric liquid polymers also may be employed as additives but normally in lower proportions not above about 10 percent by weight of the elastomeric component.

The third group of suitable film forming materials includes alloys which are physical mixtures of structurally different homopolymers or copolymers. Suitable alloys include a mixture of polyethylene and the block copolymers of the thermoplastic elastomer group. Also, polystyrene can be mixed with the block copolymers wherein a high proportion of the block copolymer will be used with a small amount of polystyrene. In addition, co-polyesters with elastomers are suitable as taught in U.S. Patent 4,389,444.

The fourth group of suitable film forming materials is a mixture of a thermoplastic elastomer and a low molecular weight (less than 3000) resin modifier. The thermoplastic elastomer is a linear or radial A-B-A block copolymer or mixture of the A-B-A copolymer with one or more simple A-B block copolymers. The A blocks are derived from styrene or styrene homologues and the B blocks are derived from conjugated dienes or lower alkenes. The low molecular weight resins generally are based on polyalphamethylstyrene, polystyrene, polyvinyltoluene and similar aromatic resins. Also included as suitable resins are copolymers thereof, coumarone indene, and related cyclic compounds. The resins are present in an amount of about 10 to about 200 parts for each 100 parts of thermoplastic elastomer. In addition to these resins, small proportions of other resins, i.e., not above about 25% by weight of the elastomeric component, may be added. These resins include hydrocarbon resins, rosin, hydrogenated rosin, rosin esters, polyterpene resins and the like. The resulting films are highly elastic and have a relatively low rubber modulus. The films generally possess high friction properties and are very flexible, extensible, soft and normally exhibit a Gurley stiffness of less than about one at a thickness of one mil.

The last group of suitable films are the pressure-sensitive adhesive films. These films include the hot melt adhesive films. The thermoplastic rubber block copolymers provide suitable formulations for pressure-sensitive adhesive film-formers which can be extruded to produce a highly desirable product. The elastomer will be of a high molecular weight and the resin a low molecular weight. A typical formulation is from 25 to 250 parts by weight of a tackifier resin in proportion to 100 parts by weight of a thermoplastic elastomeric component. Suitable tackifier resins include rosin and rosin derivatives of

the abietic acid type or the pimaric acid type. The rosin acids react with alcohols to form esters, e.g., hydrogenated glycerine esters. Hydrocarbon tackifier resins are suitable and include low molecular weight polymers containing primarily aromatic, aliphatic or diene monomers. An example of an aliphatic-monomer containing resins is one identified as Wing Tack 95 manufactured and sold by Goodyear Tire and Rubber Company. The terpene resins such as Piccolyte S-100 manufactured and sold by Hercules Chemical Company are also suitable.

Acrylate copolymers are also highly satisfactory. For example, methvlmethacrylate-butadiene-styrene provide a suitable film-forming material. Polyacrylates of a proper monomer composition are inherently pressure-sensitive without any compounding. This single component feature has some advantages over the compounded adhesives. Low molecular weight ingredients that can migrate to the surface of an adhesive coating are absent. Adhesive bond is a surface phenomenon, and minimizing the compositional variations is difficult to avoid in multiphase systems, while uniformity is more easily achieved in single component adhesives.

Polyacrylates possess some inherent properties superior to many other polymers used for pressure-sensitive adhesives. The polymer is saturated and resistant to oxidation. It is water white and does not yellow on exposure to sunlight. The resistance to oxidation surpasses that of most polymers used for pressure-sensitive adhesives, except silicones. Monomers with various functional groups can be introduced during polymerization, and an adhesive with various degrees of thermosetting properties can be prepared.

Acrylic adhesives are available as solutions, aqueous emulsion melts, and 100% reactive solids.

A third type of pressure-sensitive adhesive film involves an ethylene-vinyl acetate based system wherein a tackifying resin is present. Generally the ethylene vinyl acetate copolymer is a random copolymer containing from about 40% to about 60% vinyl acetate by weight. The ethylene vinyl acetate polymer may be used singly or as a mixture with A-B-A or A-B block copolymers. Suitable tackifying resins include rosins and hydrogenated rosins and mixtures thereof.

Suitable film-formers are discussed in the following U.S. Patents: 3,783,072; 3,932,328; 3,984,509; 4,080,348; 4,178,337; 4,379,806; 4,389,444; and 4,301,255.

The film forming composition of all groups may contain relatively small proportions of various other materials such as antioxidants, heat stabilizers and ultraviolet absorbers, release agents, extenders, plasticizers, pigments, fillers and the like.

The film-formers discussed in Groups II, IV and V can be solvent coated followed by heat lamination or pressure lamination as well as extruded. The film-formers of Group V, which are the adhesive films, can be coated onto the fabric or absorbent unit from water-based systems.

All of the film-formers discussed above can be extruded as a foam as well as a continuous film. Generally a closed cell foam is preferred so that the foam is liquid-impermeable.

Suitable extruding techniques include the single screw extrusion and coextrusion techniques, the primary object

being to provide a thin film which at least in its molten form is tacky. Of course, the pressure-sensitive adhesive films are still tacky after formation.

The absorbent unit of the present invention is substantially coextensive with the initially molten film or is smaller in size than the film. The absorbent unit is typically a fluff batt such as that used in conventional disposable diapers. Alternatively, the absorbent unit is a self-contained unit such as a compressed composite unit comprising a resilient fiber fabric, containing superabsorbent and a layer of wood pulp fibers, or the like, which has been compressed to form a highly absorbing unit. Still another form of an absorbent unit is a fabric which contains superabsorbent. All of the absorbent units are capable of absorbing at least about 5 milliliters per gram of the absorbent unit weight.

If the absorbent unit is a fluff batt, generally it is formed of hydrophilic fibers such as rayon fibers, cellulosic fibers, or peat moss, or mixtures thereof, or acrylic fibers, or the like. Cellulosic fibers include wood pulp fibers, cotton linters, and the like. Other cellulosic fibers that might be used are rayon fibers, flax, hemp, jute, ramie, cotton and the like. Combinations of fibers and fabric can be formed in many different ways so long as the absorbent unit will absorb at least about five milliliters per gram of weight of the absorbent unit. The absorbent unit preferably is from about 50 mils to about 1/2 inch in thickness. Most preferably, the absorbent unit is from about 80 mils to about 200 mils in thickness. Preferably, the absorbent unit will contain superabsorbent. The superabsorbent is present either on the resilient fibers or placed with any cellulosic fibers that are present or both and is generally a water-insoluble, water-swellable polymeric substance capable of

absorbing water in an amount which is at least 10 times the weight of the substance in its dried form.

The superabsorbent is in the form of fibers, spheres, particles, bits of film, globules, webs, film, foam or the like, or may be applied in the form of a liquid monomer solution which is subsequently polymerized. The superabsorbent prepared by polymerization of a monomer solution placed on fibers in a web is most frequently in the form of globules and bits of film-like particles in the web structure.

One type of superabsorbent material provides particles or fibers which may be described chemically as having a backbone of natural or synthetic polymers with hydrophilic groups or polymers containing hydrophilic groups being chemically bonded to the backbone or an intimate mixture therewith. Included in this class of materials are such modified natural and regenerated polymers as polysaccharides, including for example, cellulose and starch and regenerated cellulose which are modified by being carboxyalkylated, phosphonoalkylated, sulfoalkylated, or phosphorylated to render them highly hydrophilic. Such modified polymers may also be cross-linked to improve their water-insolubility.

These same polysaccharides may also serve, for example, as the backbone on to which other polymer moieties may be bonded by graft copolymerization techniques. Such grafted polysaccharides and their method of manufacture are described in U.S. Patent No. 4,105,033 to Chatterjee et al. and may be described as polysaccharide chains having grafted thereon a hydrophilic chain of the general formula:

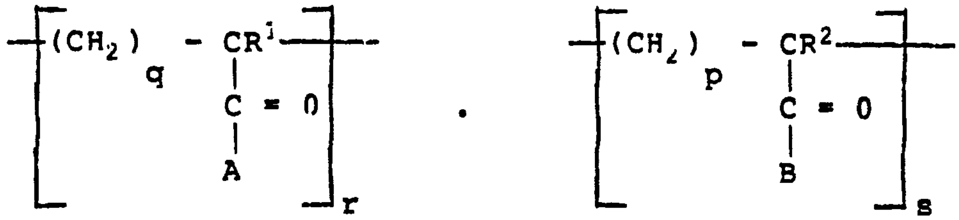

wherein A and B are selected from the group consisting of $-OR^3$, - O(alkali metal), $-OHNH_3$, $-NH_2$, wherein $R^1$, $R^2$, and $R^3$ are selected from the group consisting of hydrogen and alkylene having 1 to 4 or more carbon atoms wherein r is an integer having a value of 0 to about 5000 or more, s is an integer having a value of 0 to about 5000 or more, r plus s is at least 500, p is an integer having a value of 0 or 1, and q is an integer having a value of 1 to 4. The preferred hydrophilic chains are hydrolyzed polyacrylonitrile chains and copolymers of polyacrylamide and polysodium acrylate.

In addition to the modified natural and regenerated polymers, the hydrocolloid component may comprise wholly synthetic hydrophilic particles. Examples of those now known in the art are polyacrylonitrile fibers which may be modified by grafting moieties thereon such as polyvinylalcohol chains, polyvinyl alcohol itself, hydrophilic polyurethane, poly(alkyl phosphonates), partially hydrolyzed polyacrylamides (e.g., poly(N-N-dimethylacrylamide), sulfonated polystyrene, or a class of poly(alkyleneoxide). These highly hydrophilic synthetic polymers may be modified by other chemical treatments such as cross-linking or hydrolysis. Further examples known in the art are the non-ionic polymers such as polyoxyethylene, polyoxypropylene, and mixtures thereof which have been suitably cross-linked, either chemically or by irradiation. Still another more recent type is a derivative of isobutylene-malic and acrylate monomers, such as sodium, potassium, ammonium, (or a combination of cations), acrylate, may be

placed on the absorbing layer by spraying or otherwise placing a solution thereon, followed by polymerization and cross-linking, for example, by irradiation.

In addition, naturally occurring materials such as gums may be used. Examples of such suitable gums include guar gums, acacia gums, locust bean gums and the like.

The superabsorbent can be placed between the surface of the film and the absorbent unit so long as a sufficient amount of the surface of the absorbent unit is in contact with the initially molten film to provide substantial lamination.

A film or fabric is optionally also laminated to the initially molten film. The fabric may be on the side of the film opposite the absorbent unit in which case it is preferred that the absorbent unit be a "self-contained unit" such as that which has a fabric base. Alternatively, a liquid-permeable fabric or perforated film may be on the same side of the initially molten film as the absorbent unit covering the absorbent unit but being in contact with the film around the periphery of the absorbent unit. In addition, fabric or perforated film may be on both sides of the initially molten film. In this instance, the absorbent product which is formed has a cloth-like feel.

The method for making the absorbent article of the present invention includes bringing the absorbent unit and the fabric in contact with the initially molten film while the film is still molten (tacky). In the case of non-pressure-sensitive adhesive films, it is necessary that the film still be substantially in a hot state. However, with use of a pressure-sensitive film, the film may be made first and the fabric and absorbent unit combined with it at a later time. The methods are illustrated in

Figures 4, 5, and 6. There are basically two extrusion type processes which can be used. The first of these is what might be called a "true" extrusion wherein all of the components to form the film are placed in the extruder where they are melted, mixed and extruded through a die at pressures, temperatures, and conditions, to provide a film of 10,000 centipoises up to about 2,000,000 centipoises. Another method is used when preparing the hot melt pressure-sensitive adhesive wherein the hot melt mix is prepared first and is then placed through an extruder to form the film or is coated onto the substrate by use of a positive displacement pump and an extrusion die.

The product of the present invention is soft, pliable, thereby flexible, noiseless, and provides a substantially 100 percent stable product. Furthermore, improved absorbency and wicking ability have been observed in the product.

Another advantage of the process and product of the present invention, is the abilitv to add additional components such as elastomers in the margins to gather the product. While the initially molten film is still tacky, elastic can be placed between the film and the fabric prior to final lamination. Preferably, the elastic is stretched, placed in contact with the film, the fabric is placed over the elastic, and lamination subsequently takes place. While the elastic is still in a stretched condition, the elastic is absolutely bonded and will not become dislodged during storage or use of the absorbent product. The flexibility of the manufacturing techniques permits the easy addition of discrete elastic pieces or of discrete fabric pieces which may extend bevond the margins of the laminated product. For instance, a rectangular product consisting of initially molten film, an absorbent unit smaller than the size of the film, and a fabric unit

coextensive with the film, could have laminated between the fabric and the film in the margins pieces of fabric which extend beyond the rectangular shape of the absorbent article so as to provide a T-shape, an "I" configuration, or other designs.

Other advantages afforded by the present invention include multiple-article production lines such as that exemplified in Figure 6, high speed production, production substantially without waste, and modification of a product in the production line with ease and at minimal cost. For instance, a production line might produce disposable diapers one day, and with simple modification produce sanitary napkins the next day. The absorbent unit can be prepared off-line and consequently will not impede the speed with which the manufacturing can take place. Other components likewise, which are to be placed with the extruded initially molten film, can be prepared from other production lines and then readily combined with the initially molten film while it is still in its tacky state.

Examples of methods of preparing the absorbent structure of the present invention are as follows. These examples are not intended to be limiting in any way and extensions and modifications thereof, without departure from the spirit and scope of the invention, will be apparent to one skilled in the art.

<u>Example 1</u>

An absorbent unit is formed of polyester fibers by dry laying the fibers, i.e., by air laying or carding to form a web. Specifically, the polyester fibers contain a minor portion of fusible fibers which soften at a lower temperature than the rest of the fibers. The web is heat bonded

by passing air at a temperature of 350°F. through the web for about 10 seconds. The resulting web is 25 grams per square meter, basis weight. Thee specific polyester fibers used are identified as Type 99 Hollofil fibers manufactured and sold by E.I. Dupont Company. The fibrous web is placed on top of a sheet of wet-formed chemically delignified wood pulp fibers, the fibers being identified as RayFloc JLD manufactured by ITT Rayonair having a basis weight of 50 grams per square meter. A powdered superabsorbent polymer is uniformly sprinkled onto and into the nonwoven fiber polyester structure at a concentration of 200 grams per square meter. The particular superabsorbent used is identified as Permasorb 10 manufactured by National Starch and Chemical Corporation. The structure is sprayed with a mist of water on the polyester side and then subjected to a compression force of 640 psi for 30 seconds. On release of the pressure the structure remains compressed and is available to function as an absorbent unit.

Polyethylene pellets are placed in the feed hopper of a single screw extruder. See U.S. Patent 4,178,337. The polyethylene is identified as NA 598 manufactured and sold by U.S. Industial Chemicals Company, New York, New York. The feeder places the polyethylene in direct contact with the extruder hot screw heated to a temperature of about 350°-400°F. The molten polyethylene exiting from the extruder is a continuous sheet of about 30 mils thick which is drawn to a thickness of about one mil or a predetermined thickness in order to provide a desired weight per unit area.

While the polyethylene film is in its drawn molten state the previously prepared absorbent unit, which is narrower than the film, is placed in contact with the film and a polyester nonwoven fabric having a weight of about

0.7 oz./sq. yd. is placed over the absorbent unit and is substantially coextensive with the film. The absorbent unit and the fabric are laminated to the molten film.

Conventional adhesive tape tabs are added at each side edge of the film toward one end of the final severed diaper product.

Example 2

An absorbent unit is prepared using the same polyester fibrous web formed in Example 1, the web is coated by flooding it with an aqueous solution of 38% solids, the solution solids being 90% sodium acrylate and 10% acrylic acid. Excess solution is drained from the web. The web is then subjected to 6 megarads of electron beam radiation after which about 70 grams/$m^2$ of PSA is present. The web is again flooded, drained and irradiated to yield a total of about 140 gm/$m^2$ PSA. A third time after flooding and draining, the web is subjected to 12 megarads of electron beam radiation to polymerize and crosslink the monomer and form polysodium acrylate (PSA) affixed to the polyester fiber. Two hundred grams/$m^2$ of PSA is present in the substrate. This is equivalent to 800% dry-add-on. This coated substrate is passed beneath a hammer mill that deposits chemically treated wood pulp fibers onto the polyester web. Vacuum is applied under the polyester web so as to cause some of the pulp fibers to at least partially migrate into the polyester web and become integral therewith. The major portion of the wood pulp fibers will reside on the surface providing a layer containing wood pulp fibers of 50 gms/$m^2$. The surface of the pulp layer is sprayed with water so that the total moisture content of the pulp is 10% by weight. This structure is compressed at a level of 640 psi for 30 seconds. Upon release of pressure the pulp has formed into a high

density layer with a capillary size suitable for liquid wicking and the resilient fiber layer remains compressed. Upon use of this structure when a significant amount of liquid contacts the surface and migration of the liquid into the product takes place, the superabsorbent becomes soft and releases the resilient fibers so that the thickness of the absorbent structure increases markedly. This provides an area for storage of liquid wherein the capillary size is large.

Particulate components for an elastomeric extrudable film are placed in the feed hopper of an extruder. These components are 97 parts by weight of a styrene-isoprene-styrene elastomer Kraton 1107, 2 parts butyl zimate and 1 part Santovar. Kraton 1107 copolymer is a thermoplastic elastomeric A-B-A (styrene-isoprene-styrene) block copolymer offered by the Shell Chemical Company, wherein the styrene content (that of the A blocks) is about 12-15%, closer to 15% by weight of the block copolymer and the polymer possesses a solution viscosity of about 2,000 centipoises at 25% solids in toluene at room temperature (using a Brookfield Viscometer with a No. 4 spindle at 60 r.p.m.), and a number average molecular weight of about 110,000-125,000. The butyl zimate and Santovar are antioxidants.

A thin film, about 2 mils thick, is extruded and while the film is in a molten state the absorbent unit and a spunbonded polypropylene nonwoven facing fabric is placed over the absorbent unit, as in Example 1, and the product is laminated. As before, appropriate tape tabs are affixed to the side edges of the product. The resulting diaper product weighs about 35 grams and holds at least about 150 mls. of liquid.

Example 3

An absorbent unit is formed wherein the same polyester web is treated with a wood pulp fiber-water slurry which is drained through the polyester fiber web so that a pulp deposit of 50 grams per square meter is formed on one side of the polyester web. The two layered sheet is dried. Onto the polyester web side of the sheet is sprayed the same monomer solution as in Example 2, so that practicallv no monomer solution contacts the wood pulp fiber layer. As before, the sample is coated and treated three times providing 800% dry-add-on of PSA. The resulting structure at a moisture content of approximately 50% by weight is compressed at a level of 640 psi for 30 seconds. Upon release of the pressure the structure remains compressed and is ready for use as taught hereinbefore.

The moisture level of the two layers prior to compression preferably is sufficient to make the exterior surface of the superabsorbent tacky so as to provide a temporary bonding of the wet resilient fibers under compression. Thus, the compressed composite structure remains compressed until it is in contact with sufficient liquid for the superabsorbent to begin swelling and to thereby release the bonds formed with the resilient fibers.

The amount of superabsorbent added to the absorbing layer should not exceed the volumetric displacement afforded by the subsequent compression.

The structure generally is compressed sufficiently to reduce the thickness of the structure by at least 50% and the pressure is sufficient to cause the composite to remain compact after the pressure is released. Compression should not be high enough to substantially crimp or crease the fibers in the absorbing layer.

The prepared absorbent unit is combined with a molten film alloy consisting of 42 parts by weight poly(ethylene terephthalate coethylene azelate) and 58 parts by weight Kraton 1107 discussed in Example 2. The combining of the absorbent unit and a facing fabric, such as polyester, is accomplished in the same manner as in Example 2.

Example 4

Chemically treated wood pulp fibers, such as Rayfloc J, are laid on a surface to form a loosely compacted cellulosic fibrous batt. Such batts are used in conventional disposable diapers as the absorbent unit. Such batts are placed on a conveyor and individually come in contact and are laminated to a molten film.

The film is formed by an extruder. The film constituents are 40 parts by weight Kraton 1107 and 60 parts by weight polyalphamethylstyrene (Amoco 18-290, manufactured and sold by Amoco Chemical Co.). As the film is extruded, a polyester nonwoven fabric (about 0.7 oz./sq. yd.) is placed in contact with the film on one side while simultaneously the absorbent batt and another layer of polyester are placed on the opposite side of the film. The polyester covers the absorbent batt on the side of the film where the batt contacts the film. As discussed heretofore, a suitable closure is added and the film severed to cut the line of product into individual units and, if desired, into specific shapes along the side or end edges. (See Fig. 1)

The resulting product has a fabric feel on both sides and holds up to at least 150 mls of body exudate. The absorbent batt is substantially, completely, stabilized and hence, even during use does not break apart or become lumpy in the crotch. Furthermore, the product wicks

liquid well from one portion of the batt to another. Thus, the liquid holding capacity is substantially, fully, utilized in the product.

Example 5

In this example, pressure-sensitive adhesive films are extruded or applied by hot melt coating techniques to provide a product to which components can be added after the film is no longer molten. To make a diaper product a soft fabric is laminated to one side of the film. On the other side of the film an absorbent unit covered with a liquid-permeable fabric or film is laminated to the film. The fabric is substantially, co-extensive with the film whereas the absorbent is smaller, preferably around the entire periphery, than the film. Another advantage of the pressure sensitive adhesive (PSA) film is that it eliminates the necessity of adding a closure to the product. For instance, the product of Figure 1 shows the film exposed on the diaper "ears" to provide a PSA closure. Also in Figure 3, a sanitary napkin is depicted which for the securing means shows a portion of PSA film exposed on the underside to provide securement to the clothing of the user.

Two suitable formulations for PSA film appear below:

TABLE I

| | Kraton 1107 | Wing Tack 95 | Butyl Zimate | Santovar A |
|---|---|---|---|---|
| Sample A | 100 | 80 | 2 | 1 |
| Sample B | 100 | 140 | 2 | 1 |

In Table I above, all parts are given in parts by weight. Sample A is a PSA film which is extruded onto a substrate or onto a conveyor after which other components are added later. Sample B may be applied by extrusion or by hot melt coating techniques.

The foregoing is intended as illustrative of the present invention but is not intended as limiting in any way. Numerous variations and modifications may be made without departing from the true spirit and scope of the invention.

Claims

1. An absorbent structure comprising a laminate of an initially molten film and an absorbent unit, said absorbent unit being capable of absorbing at least about five milliliters per gram of body exudate.

2. The absorbent structure of Claim 1 wherein said laminate additionally comprises a fabric on at least one side of said film substantially co-extensive with said film.

3. The absorbent structure of Claim 1 or claim 2 wherein said initially molten film is a plastic film.

4. The absorbent structure of Claim 3 wherein said plastic film is selected from the group consisting of poly-ethylene, ethylenevinyl acetate, polypropylene, polystyrene, polyurethane, polyvinyl chloride, polybutylene, copolyesters, vinyls, acrylics and mixtures thereof.

5. The absorbent structure of Claim 1 orclaim 2 wherein said initially molten film is a thermoplastic elastomeric film.

6. The absorbent structure of Claim 5 wherein said thermoplastic elastomeric film is selected from the group consisting of ethylenevinyl acetate, polyurethane, polyolefin blends, copolyesters, block copolymers and blends thereof.

7. The absorbent structure of Claim 1 orclaim 2 wherein said initially molten film is a film alloy of structurally different homopolymers or copolymers.

8. The absorbent structure of claim 1 or claim 2 wherein said initially molten film is a mixture of a thermoplastic elastomer and a low molecular weight resin modifier.

9. The absorbent structure of claim 1 or claim 2 wherein said initially molten film is a pressure sensitive adhesive film.

10. The absorbent structure of claim 9 wherein said pressure sensitive adhesive is a hot melt adhesive.

11. The absorbent structure of claim 1 or claim 2 wherein said initially molten film is a foam.

12. The absorbent structure of any one of claims 1 to 11 wherein said absorbent unit is capable of absorbing at least about 150 milliliters of urine.

13. The absorbent structure of any one of claims 1 to 12 wherein said absorbent unit is a loosely compacted cellulosic fibrous batt.

14. The absorbent structure of any one of claims 1 to 13 wherein said absorbent unit is a self-contained compressed composite unit.

15. The absorbent structure of any one of claims 1 to 14 wherein said absorbent unit contains superabsorbent.

16. The absorbent structure of any one of claims 1 to 15 wherein said absorbent unit is comprised of fabric and superabsorbent.

17. The absorbent structure of claim 2 or any claim dependent thereon wherein said fabric is on the same side of said film as said absorbent unit and is a liquid-permeable fabric covering said absorbent unit.

18. The absorbent structure of claim 17 wherein a further fabric is laminated to the side of said film opposite said absorbent unit.

19. The absorbent structure of claim 2 or any claim dependent thereon wherein said first fabric is a perforated film.

20. The absorbent structure of claim 17 or any claim dependent thereon wherein elastic members are laminated between said fabric and said film on each side of said absorbent unit.

21. A disposable diaper comprising the absorbent structure of any one of claims 1 to 20 and a closure means for securing said diaper about the waist of the wearer.

22. A sanitary napkin comprising the absorbent structure of any one of claims 1 to 20.

23. The sanitary napkin of claim 22 when dependent on claim 2 or any claim dependent thereon wherein said fabric envelopes said absorbent unit and said film.

24. A corrugated disposable diaper comprising (a) a liquid-permeable facing; (b) an absorbent unit; (c) an initially molten liquid-impermeable film; (d) elastic corrugating means comprising multiple strands of stretched elastic placed transverse to the longitudinal axis of said diaper; (e) a fabric contacting said initially molten film on the side of said elastic corrugating means, said corrugating means being sandwiched between said film and said fabric, said unit being smaller in dimension than said film to provide margins of said film being exposed surrounding said unit, said facing being coextensive with and laminated to said film in said margins.

25. The disposable diaper of Claim 24 wherein said elastic corrugating means is present in the central portion of said diaper.

26. The disposable diaper of claim 24 or claim 25 wherein said elastic corrugating means is present in at least one waist portion of said diaper.

27. A method for preparing an absorbent structure comprising:

combining a molten film with an absorbent unit and a liquid permeable fabric; and

laminating said film, absorbent unit and fabric said fabric substantially covering at least one side of said film and being coterminous with said film.

FIG-1

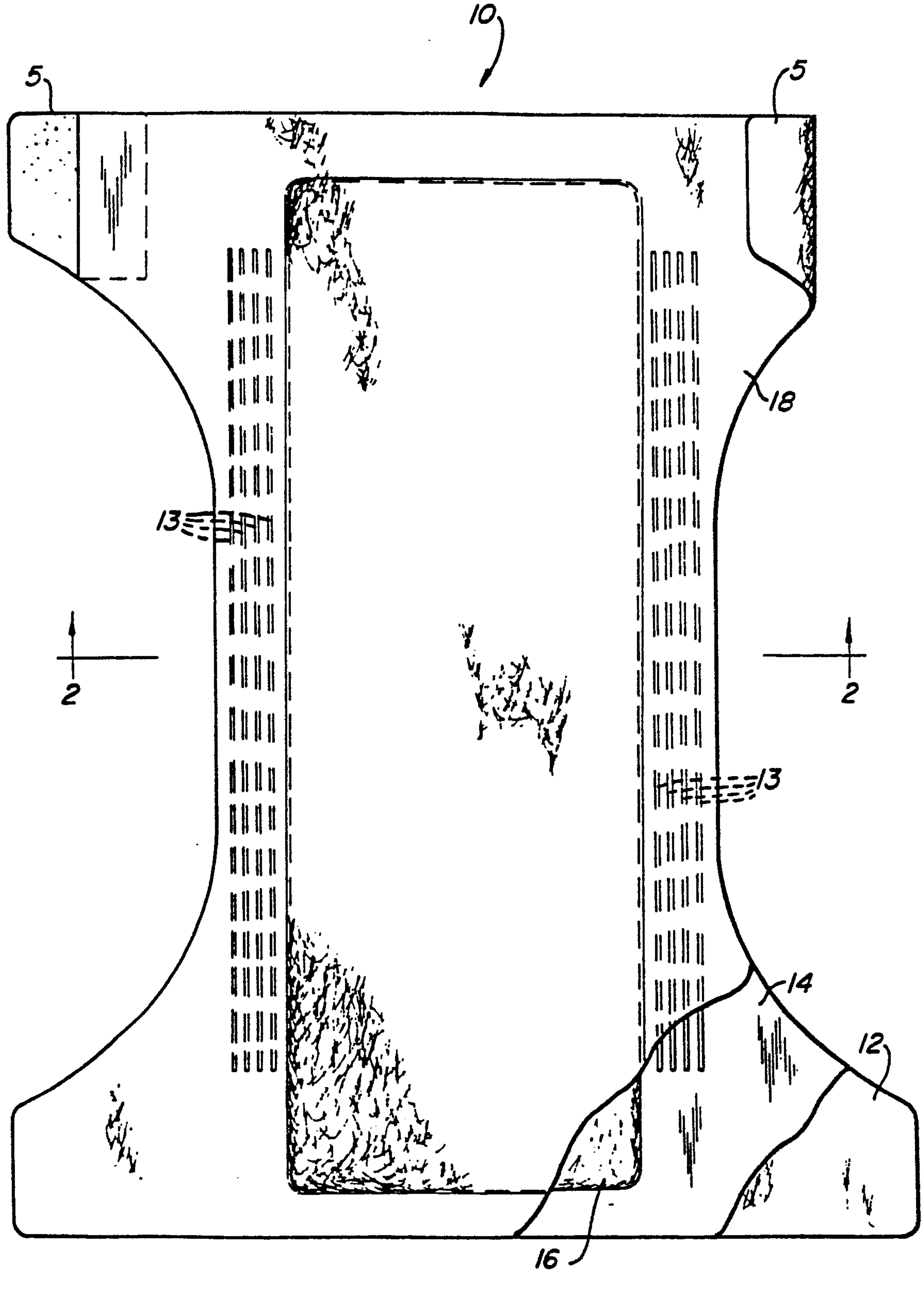

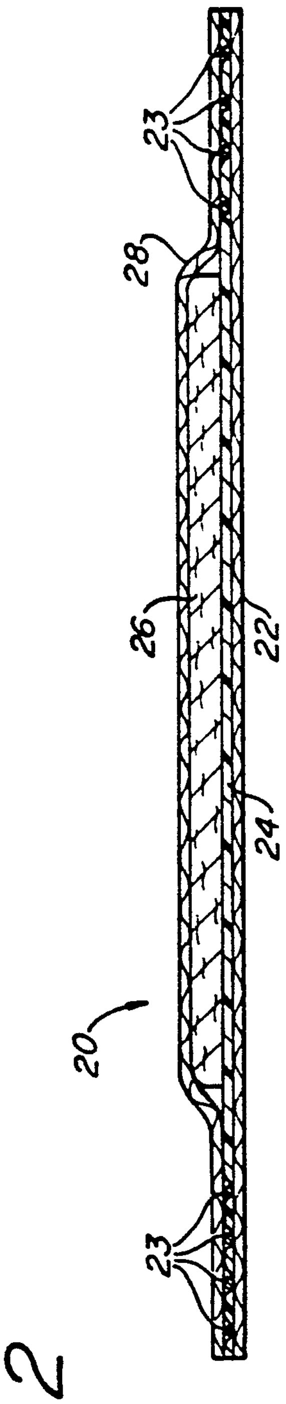


FIG-2

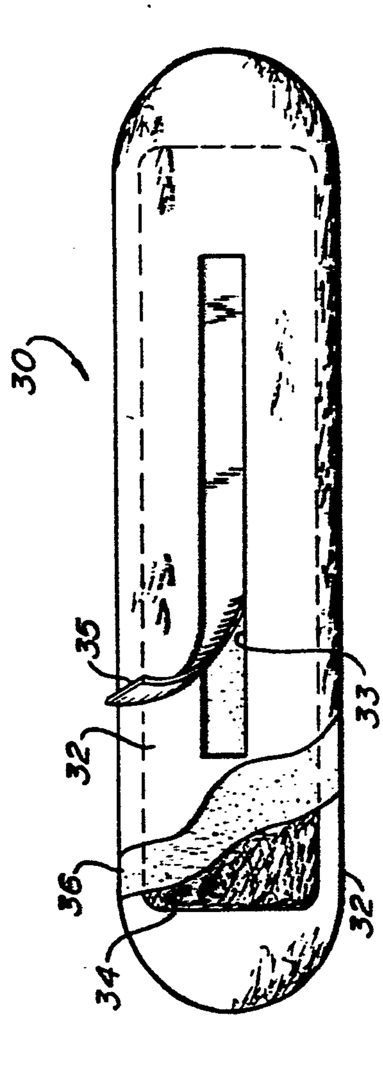


FIG-3

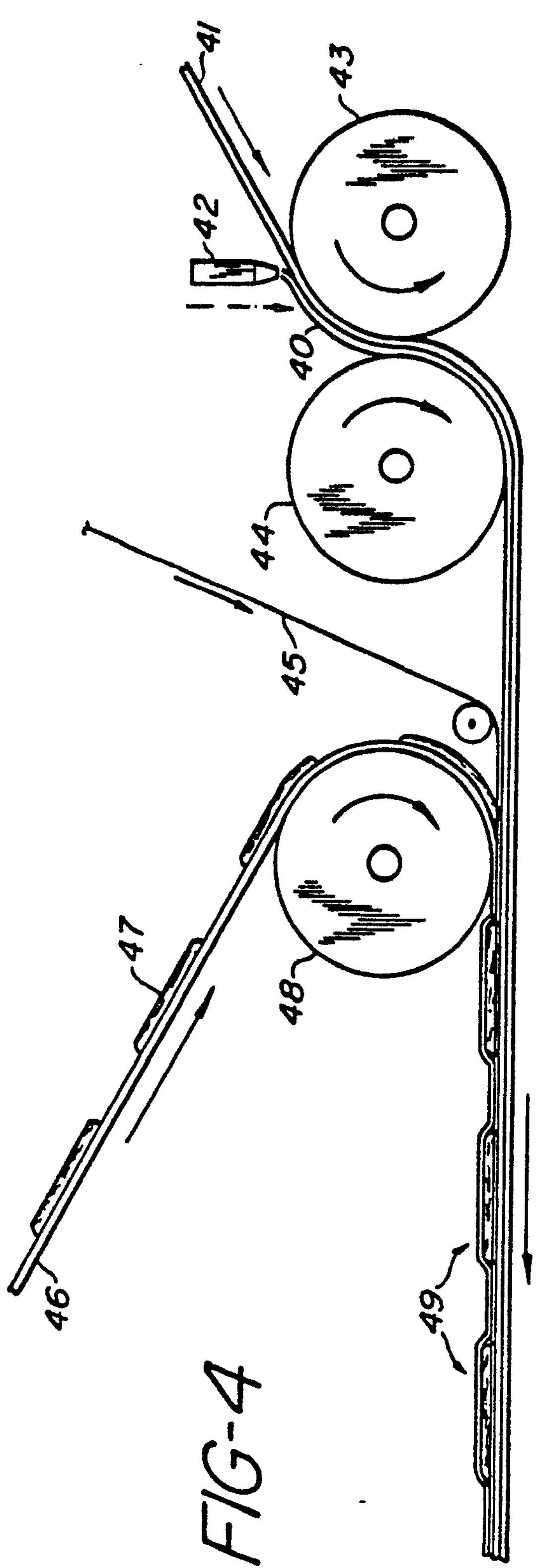


FIG-4

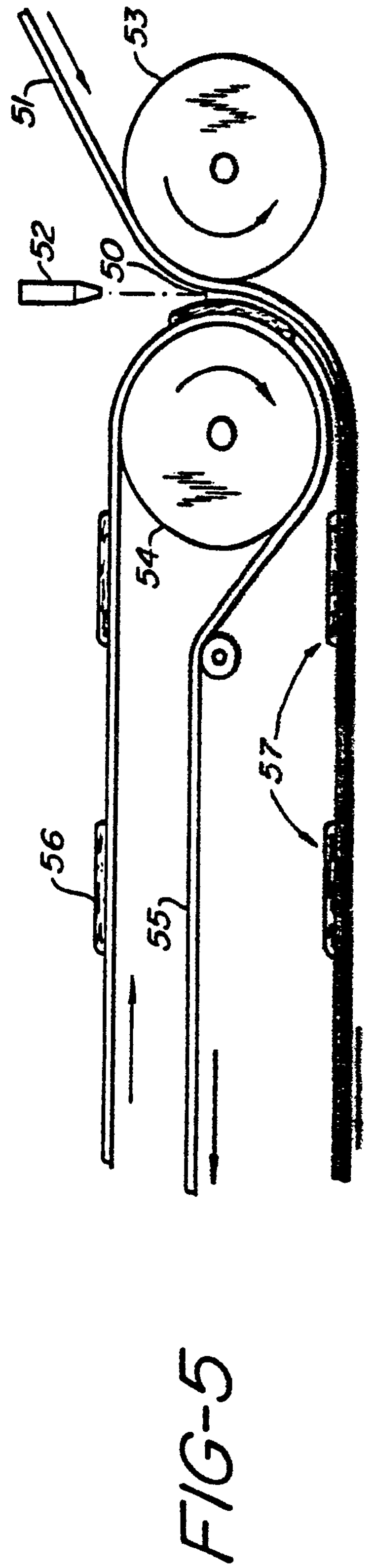


FIG-5

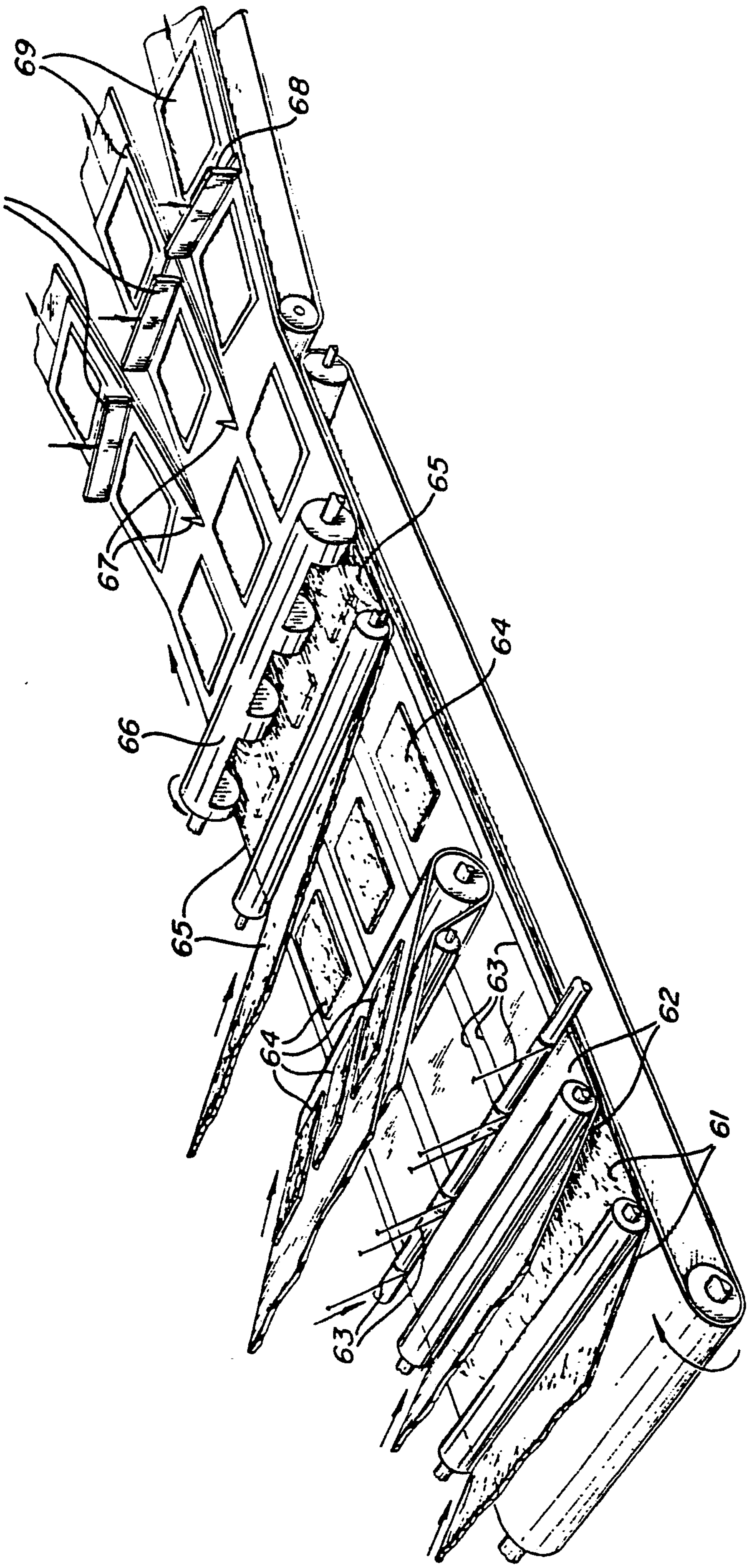


FIG-6

FIG-7

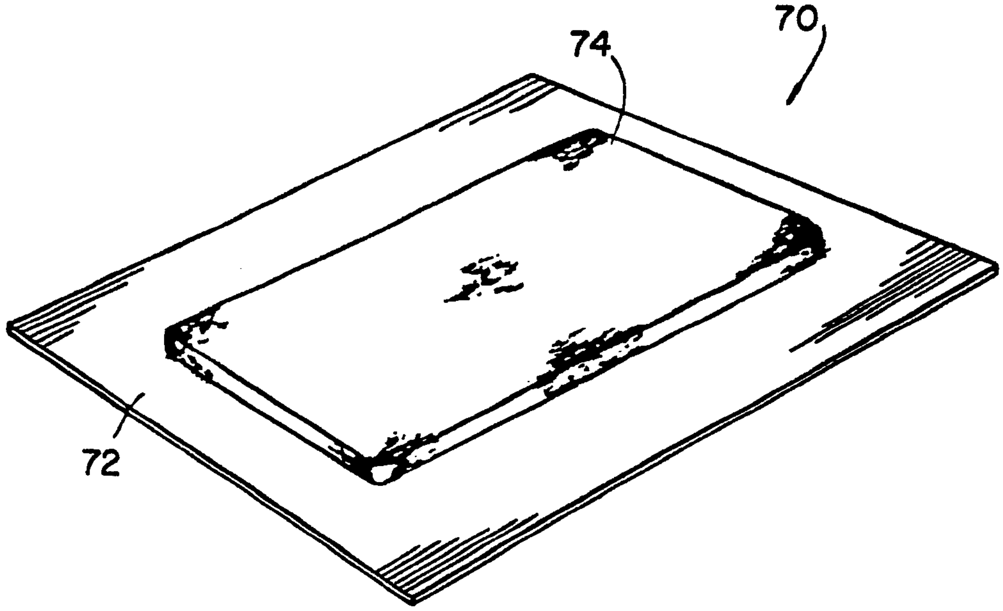


FIG. 8

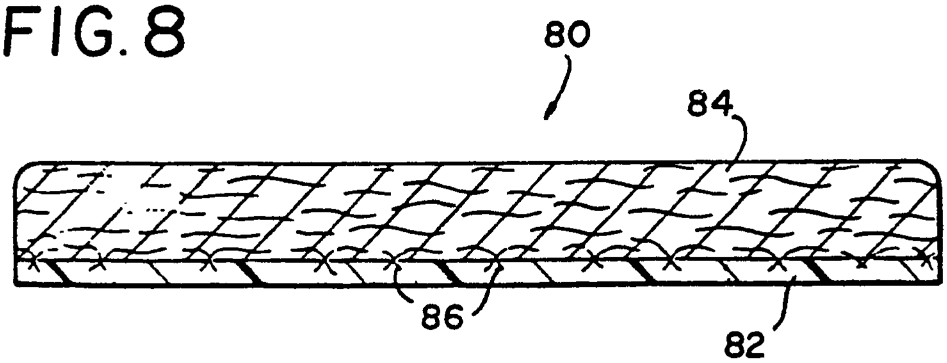

FIG.-9

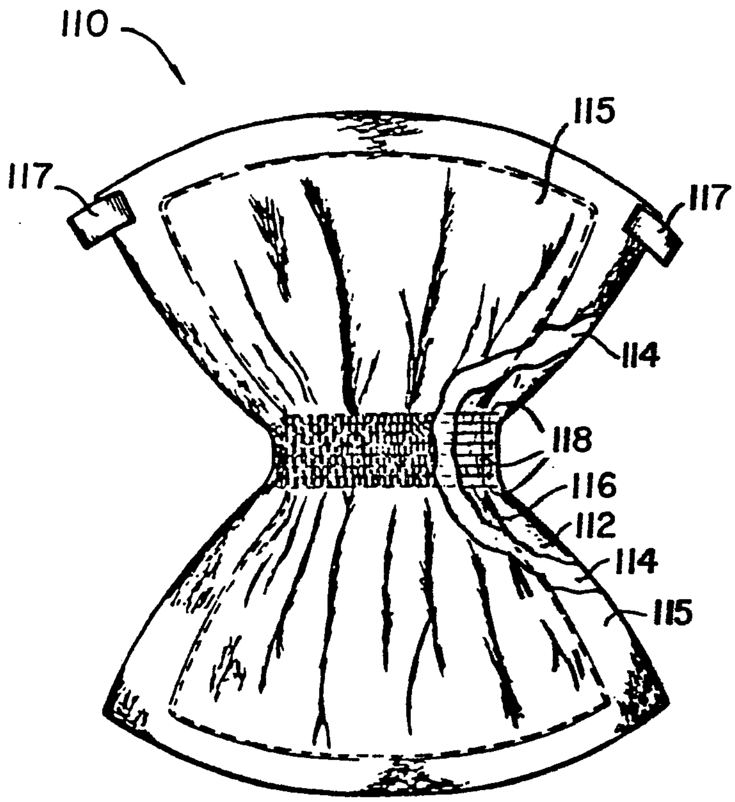

FIG.-10

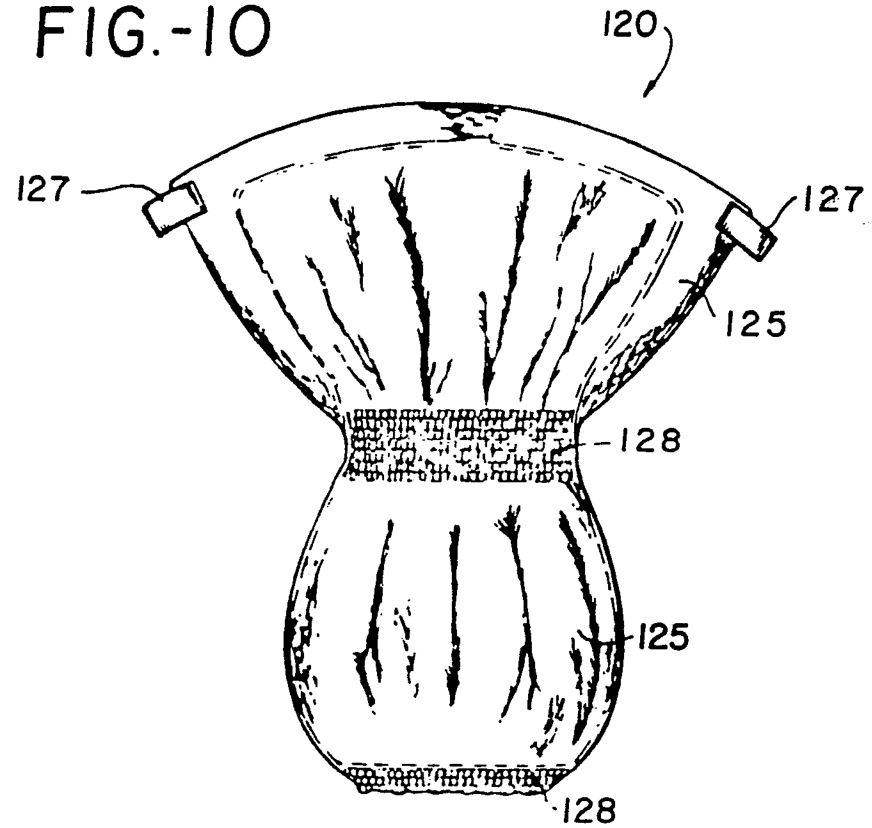


FIG.-11